(19) 

(11) **EP 3 365 493 B1**

(12) **EUROPEAN PATENT SPECIFICATION**

(45) Date of publication and mention of the grant of the patent: **12.08.2026 Bulletin 2026/33**

(21) Application number: **16784500.7**

(22) Date of filing: **20.10.2016**

(51) International Patent Classification (IPC): ***D21C 3/00*** *(2006.01)* ***D21C 3/24*** *(2006.01)* ***D21C 3/26*** *(2006.01)* ***C12N 9/42*** *(2006.01)*

(52) Cooperative Patent Classification (CPC): **D21C 3/006; D21C 3/24; D21C 3/26; D21C 5/005**

(86) International application number: **PCT/EP2016/075241**

(87) International publication number: **WO 2017/068048 (27.04.2017 Gazette 2017/17)**

(54) **USE OF CELLULASE TO IMPROVE VISCOSITY CONTROL OF DISSOLVING PULP**

VERWENDUNG VON CELLULASE ZUR VERBESSERUNG DER VISKOSITÄTSKONTROLLE VON KRAFTAUFLÖSUNG VON ZELLSTOFF

UTILISATION DE CELLULASE POUR AMÉLIORER LA RÉGULATION DE LA VISCOSITÉ DE LA PÂTE DE DISSOLUTION KRAFT

(84) Designated Contracting States: **AL AT BE BG CH CY CZ DE DK EE ES FI FR GB GR HR HU IE IS IT LI LT LU LV MC MK MT NL NO PL PT RO RS SE SI SK SM TR**

(30) Priority: **23.10.2015 PCT/EP2015/191244**

(43) Date of publication of application: **29.08.2018 Bulletin 2018/35**

(73) Proprietor: **Novozymes A/S**
**2880 Bagsvaerd (DK)**

(72) Inventors:
- **LOUREIRO, Pedro, Emanuel, Garcia**
  **2880 Bagsvaerd (DK)**
- **LUND, Henrik**
  **2880 Bagsvaerd (DK)**

(74) Representative: **NVS EPO Representatives**
**Krogshoejvej 36**
**2880 Bagsvaerd (DK)**

(56) References cited:
**WO-A1-2009/147210**
**WO-A1-2012/089024**
**WO-A1-2013/039776**
**WO-A1-2013/171364**
**WO-A1-2014/041251**
**WO-A2-2010/078930**
**CN-A- 104 452 397**
**US-A1- 2015 107 789**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### FIELD OF THE INVENTION

The present invention relates to treatment of partially bleached or alkaline extracted dissolving pulp with one or more cellulases. The cellulase treatment results in improved viscosity control and/or reduced viscosity of the final dissolving pulp.

### BACKGROUND OF THE INVENTION

Commercial dissolving pulp or dissolving-grade pulp is a chemical bleached pulp with a high cellulose content enough to be suitable for the production of regenerated cellulose and cellulose derivatives. Commercial dissolving pulp has special properties, such as a high level of brightness and uniform molecular-weight distribution. Commercial dissolving pulp is manufactured for uses that require a high chemical cellulose purity, and particularly low hemicellulose content, since the chemically similar hemicellulose can interfere with subsequent processes. Dissolving pulp is so named because it is not made into paper, but dissolved either in a solvent or by derivatization into a homogeneous solution, which makes it completely chemically accessible and removes any remaining fibrous structure. Once dissolved, it can be spun into textile fibers such as viscose or Lyocell, or chemically reacted to produce derivatized celluloses, such as cellulose triacetate, a plastic-like material formed into fibers or films, or cellulose ethers such as methyl cellulose, used as a thickener.

Conventional viscose manufacturing which uses dissolving pulps as raw material requires improvement with respect to its environmental impact as well as its production costs. The present invention provides a cellulase-based solution that improves the viscosity control in the production of bleached dissolving pulp, e.g., kraft and sulfite dissolving pulp. Furthermore, the reactivity of the final dissolving pulp is improved, thereby reducing the amount of chemicals used in the viscose production process and/or improving the processability in terms of viscose dope filterability in the viscose making process. Savings in the amount of chemicals utilized in the production of regenerated cellulose such as carbon disulfide ($CS_2$) in the viscose making process will reduce costs and the environmental impact.

Although it has been previously demonstrated that a cellulase can be used to decrease the pulp viscosity and/or increase dissolving pulp reactivity, such previous studies were based on the treatment of bleached dissolving pulps or commercial dissolving pulps. The present invention demonstrates that a cellulase can be applied instead earlier in the dissolving pulp production process in order to improve the viscosity control during the production process of the dissolving pulp by allowing a more precise control of the pulp viscosity along the process. This improvement in the viscosity control allows the production of a lesser amount of pulp outside the viscosity specification target and the possibility of a significant reduction of the amount of required chemicals that are traditionally used to control the pulp viscosity in the bleaching plant (e.g. NaOCl, $O_2$, $O_3$, $H_2O_2$, etc.). According to the present invention, the cellulase can be utilized either as a viscosity control aid being applied to an unbleached or partially bleached dissolving kraft pulp or it can be applied in one or in two steps as the key viscosity control stages in the fiberline after the cooking process. Moreover, the present invention surprisingly demonstrates that by applying the cellulase in the beginning or within the bleaching process, the reactivity of the final dissolving pulp is still improved. This means that the cellulase does not need to be necessarily introduced as a pre-activation step before viscose making either as a last stage in the dissolving pulp production process or in the beginning of the viscose making process.

US2015 107789 describes a process having a fractionation step immediately after kraft pulping and a fraction coming out of the fractionation called "fibers" which is treated with one or more cellulases followed by bleaching and a xylanase treatment step.

### SUMMARY OF THE INVENTION

The invention provides a method for production of dissolving pulp with reduced viscosity comprising the steps of

i) treating partially bleached or alkaline extracted dissolving pulp with a one or more cellulases (X stage) and
ii) bleaching of partially bleached/alkaline extracted pulp and
iii) optionally performing Alkaline Extraction of partially bleached/alkaline extracted pulp and thereby generating dissolving pulp with reduced viscosity,

wherein the partially bleached or alkaline extracted dissolving pulp is wood pulp from softwood trees and/or hardwood trees; and
wherein step i) is performed prior to step ii).

**DEFINITIONS**

Dissolving pulp: Dissolving pulp is a high-grade cellulose pulp, with low contents of hemicellulose, lignin and resin. This pulp has special properties, such as high level of brightness and uniform molecular weight distribution. It is used to make products that include rayon and acetate textile fibers, cellophane, photographic film and various chemical additives. To a large extent, use of dissolving wood pulp depends on its purity (cellulose content), which depends mainly on the production process. To obtain products of high quality, these so-called "special" pulps must fulfill certain requirements, such as high cellulose content, low hemicellulose content, a uniform molecular weight distribution, and high cellulose reactivity. Most of the commercial dissolving pulps accomplish these demands to a certain extent. Nevertheless, achieving high cellulose accessibility as well as solvent and reagent reactivity is not an easy task due to the compact and complex structure presented by the cellulose. About 77% of all dissolving pulp is used in the manufacture of cellulosic fibers (rayon and acetate).

Two basic processes are used to produce dissolving pulp: (a) the sulfite process; and b) the sulfate process (kraft).

To manufacture disolving-grade pulps, removing hemicelluloses from the wood fiber is crucial, because hemicelluloses can affect the filterablility of viscose, the xanthation of cellulose and the strength of the end product during the production of viscose. Hemiceluloses are removed during the cooking of wood and the subsequent bleaching.

In sulfite pulping, the acidic conditions used are responsible for removing most of the hemicellulose while in sulfate/kraft process usually a prehydrolysis step is required to remove hemicelluloses.

Another method to remove hemicelluloses is by treatment of pulps with enzymes that react only with the hemicellulose portion of the pulp.

Kraft dissolving pulp: "Kraft dissolving pulp" is synonymous with "sulphate dissolving pulp". A preferred example is a prehydrolysis kraft dissolving pulp. Kraft dissolving pulp is produced by digesting wood chips at temperatures above about 120°C with a solution of sodium hydroxide and sodium sulfide. Some kraft pulping is also done in which the sodium sulfide is augmented by oxygen or anthraquinone. As compared with soda pulping, kraft pulping is particularly useful for pulping of softwoods, which contain a higher percentage of lignin than hardwoods. The term "kraft dissolving pulp" is synonymous with "kraft dissolving cellulose" and "kraft dissolving-grade pulp" and refers to pulp that has a high cellulose content. The cellulose content of the kraft dissolving pulp is preferably at least 90% (weight/weight) such as at least 91%, at least 92%, at least 93%, at least 94%, at least 95%, at least 96%, at least 97%, at least 98% or at least 99% (w/w). Kraft dissolving pulp is manufactured for uses that require a high chemical purity, and particularly low hemicellulose content. The hemicellulose content of the dissolving pulp is preferably less than 10% (weight/weight) such as less than 9%, less than 8%, less than 7%, less than 6%, less than 5%, less than 4%, less than 3%, less than 2% or less than 1% (w/w). Kraft dissolving pulp can e.g. be used for generation of regenerated cellulose or for generation of cellulose derivatives. "Kraft dissolving-grade pulp" can also be defined as pulp that has been purified sufficiently for use in the production of viscose rayon, cellulose ethers, or cellulose esters with organic or inorganic acids.

Sulfite dissolving pulp: The sulfite process produces wood pulp which is almost pure cellulose fibers by using various salts of sulfurous acid to extract the lignin from wood chips in large pressure vessels called digesters. The salts used in the pulping process are either sulfites ($SOs^{2-}$), or bisulfites ($HSO_3$-), depending on the pH. The counter ion can be sodium (Na+), calcium ($Ca^{2+}$), potassium ($K^+$), magnesium ($Mg^{2+}$) or ammonium ($NH^{4+}$).

Sulfite pulping is carried out between pH 1.5 and 5, depending on the counterion to sulfite (bisulfite) and the ratio of base to sulfurous acid. The pulp is in contact with the pulping chemicals for 4 to 14 hours and at temperatures ranging from 130 to 160 °C (266 to 320 °F), again depending on the chemicals used.

Most of the intermediates involved in delignification in sulfite pulping are resonance-stabilized carbocations formed either by protonation of carbon-carbon double bonds or acidic cleavage of ether bonds which connect many of the constituents of lignin. It is the latter reaction which is responsible for most lignin degradation in the sulfite process. The sulfite process is not expected to degrade lignin to the same extent that the kraft process does and the lignosulfonates from the sulfite process are useful byproducts.

The spent cooking liquor from sulfite pulping is usually called brown liquor, but the terms red liquor, thick liquor and sulfite liquor are also used (compared to black liquor in the kraft process). Pulp washers, using countercurrent flow, remove the spent cooking chemicals and degraded lignin and hemicellulose.

"Bleaching" is the removal of color from pulp, primarily the removal of traces of lignin which remains bound to the fiber after the primary pulping operation. Bleaching usually involves treatment with oxidizing agents such as chlorine (C-stage), chlorine dioxide (D-stage), oxygen (O-stage), hydrogen peroxide (P-stage), ozone (Z-stage) and peracetic acid (Paa-stage) or a reducing agent such as sodium dithionite (Y-stage). There are chlorine ($Cl_2$; C-stage) free processes such as the elemental chlorine free (ECF) bleaching where chlorine dioxide ($ClO_2$; D-stage) is mainly used and typically followed by an alkaline extraction stage. Totally chlorine free (TCF) bleaching is another process where mainly oxygen-based chemicals are used. The pulp bleaching process thus typically comprise a sequence of bleaching steps with washing in between them to remove the degradation products arising from the bleaching reactions.

Cold Caustic Extraction (CCE) : A cold alkali extraction, also called Cold Caustic Extraction (CCE), is a method used to to remove short-chain noncellulosic carbohydrates (cellulose purification) that is based on physical effects such as swelling and solubilization. Usually, a CCE stage takes place at temperatures below 45°C and using very high NaOH dosage that, in the liquid phase, can reach values up to 100 g/L. Depending on the pulp consistency in use, this will determine the amount of NaOH per dry weight of pulp. Typical conditions for a CCE-stage can be 5-10% w/w NaOH in the liquid phase for at least 10 min.

Hot Caustic Extraction (HCE): the term "Hot Caustic Extraction" (HCE) is synonymous with "hot alkali extraction". HCE is a method to remove short chain hemicellulose and amorphous cellulose in pulps. A hot caustic extraction (HCE)-stage is a purification process that is based on chemical reactions, in particular alkaline peeling of hemicelluloses, which is carried out at higher temperatures and lower NaOH concentration compared to CCE.

ISO Brightness: ISO Brightness is defined in ISO 2470-1 (method for measuring ISO brightness of pulps, papers and boards), it is the intrinsic radiance [reflectance] factor measured with a reflectometer having the characteristics described in ISO 2469.

Pulp viscosity: is measured by dissolving the pulp in a suitable cellulose solvent such as in cupri-ethylenediamine (CED) and measuring the solution viscosity. This measurement gives an indication of the average degree of polymerization of the cellulose. This property can be referred as intrinsic viscosity in mL/g and measured according to ISO 5351 or as TAPPI viscosity in cP and measured according to TAPPI T 230.

Unbleached or partially bleached or alkaline extracted kraft dissolving pulp: is produced by a kraft based cooking process such as pre-hydrolysis kraft (PHK) cooking but not fully bleached and purified until becoming a commercial kraft dissolving pulp and thus it is not a finished product. Typically with a ISO brightness below 90% (such as below 85%, such as below 80%, such as below 75%, such as below 70%, such as below 65%, such as below 60%, such as below 55%, such as below 50%, such as below 45%, such as below 40%, such as below 35%, and such as below 30%).

Unbleached or partially bleached or alkaline extracted sulfite dissolving pulp: is produced by a sulfite based cooking process but not fully bleached and purified until becoming a commercial sulfite dissolving pulp and thus it is not a finished product. Tipically with a ISO brightness below 90% (such as below 85%, such as below 80%, such as below 75%, such as below 70%, such as below 65%, such as below 60%, such as below 55%, such as below 50%, such as below 45%, such as below 40%, such as below 35%, and such as below 30%).

## BRIEF DESCRIPTION OF DRAWINGS

Fig. 1 shows intrinsic viscosity of X-treated pulps versus X-D-Ep-D-P bleached pulps.
Fig. 2 shows intrinsic viscosity time and dosage profiles during the cellulase treatment (X-stage) applied to (A) an unbleached hardwood PHK pulp and (B) to a D-Ep partially bleached hardwood PHK pulp.

## DETAILED DESCRIPTION OF THE INVENTION

The invention relates to a method for production of dissolving pulp - sulfite pulp and/or kraft pulp - with reduced viscosity comprising the steps of

i) treating partially bleached or alkaline extracted dissolving pulp with a one or more cellulases (X stage) and

ii) bleaching of partially bleached/alkaline extracted pulp and

iii) optionally performing Alkaline Extraction of partially bleached/alkaline extracted pulp and

thereby generating dissolving pulp with reduced viscosity (compared to dissolving pulp obtained by the same process where the cellulase treatment is omitted),

wherein the partially bleached or alkaline extracted dissolving pulp is wood pulp from softwood trees and/or hardwood trees; and

wherein step i) is performed prior to step ii).

Special alkaline purification treatments such as HCE or CCE treatments can yield higher cellulose levels in sulfite and kraft processes. In the case of sulfite pulps, HCE is typically employed to further purify the pulp after the sulfite cooking. This additional alkaline extraction step brings unexpectedly an additional significant improvement in terms of lowering the

viscosity of the sulfite dissolving pulp when a cellulase treatment is employed as the next step. In fact, the response of unbleached sulfite pulps to enzymatic viscosity reduction is modest when compared to the use of unbleached kraft pulps. However, it was notably found that the use of a prior alkaline step as the HCE-stage improves significantly the performance of the enzymes on pulp viscosity reduction which can be linked to an improved accessibility of the enzymes to the cellulose mollecules in the sulfite pulp.

In one embodiment step ii) is performed using one or more chemicals selected from the group consisting of $ClO_2$, $O_2$, $O_3$, $H_2O_2$ and NaOCl. Step iii) is preferably an E, HCE or CCE stage. More than one bleaching steps (such as 2, 3, 4 or 5) can be performed. Likewise more than one alkaline extraction (such as 2, 3, 4 or 5) can be performed.

In a preferred embodiment, step i) is performed after step iii). In a further preferred embodiment, step i) is performed before and after step ii) and in an additional preferred embodiment, step i) is performed before and after step iii).

In one embodiment the one or more cellulases used in step i) has a sequence identity of at least 60% [such as at least 65%, such as at least 70%, such as at least 75%, such as at least 80%, such as at least 85%, such as at least 90%, such as at least 95%, such as at least 99%] to SEQ ID NO: 1. In a preferred embodiment the one or more cellulase used in step i) is SEQ ID NO: 1.

In one embodiment the one or more cellulases used in step i) has a sequence identity of at least 60% [such as at least 65%, such as at least 70%, such as at least 75%, such as at least 80%, such as at least 85%, such as at least 90%, such as at least 95%, such as at least 99%] to SEQ ID NO: 2. In a preferred embodiment the one or more cellulase used in step i) is SEQ ID NO: 2.

In one embodiment the one or more cellulases used in step i) has a sequence identity of at least 60% [such as at least 65%, such as at least 70%, such as at least 75%, such as at least 80%, such as at least 85%, such as at least 90%, such as at least 95%, such as at least 99%] to SEQ ID NO: 3. In a preferred embodiment the one or more cellulase used in step i) is SEQ ID NO: 3.

The concentration of the one or more cellulases used in step i) is preferably from 0.05 mg/kg oven dry pulp to 100 mg/kg oven dry pulp, such as from 0.05 mg/kg oven dry pulp to 1 mg/kg oven dry pulp, for example from 1 mg/kg oven dry pulp to 2 mg/kg oven dry pulp, such as from 2 mg/kg oven dry pulp to 5 mg/kg oven dry pulp, for example from 5 mg/kg oven dry pulp to 10 mg/kg oven dry pulp, such as from 10 mg/kg oven dry pulp to 20 mg/kg oven dry pulp, for example from 20 mg/kg oven dry pulp to 40 mg/kg oven dry pulp, such as from 40 mg/kg oven dry pulp to 60 mg/kg oven dry pulp, for example from 60 mg/kg oven dry pulp to 80 mg/kg oven dry pulp, or such as from 80 mg/kg oven dry pulp to 100 mg/kg oven dry pulp, or any combination of these intervals.

The method according to the invention results in an improved viscosity control, thereby allowing the reduction in the production of dissolving pulp outside final viscosity specification, typically more than 50% (such as more than 60% or more than 70%) reduction in the production of off-grade dissolving pulp with respect to viscosity. In one embodiment the method according to the invention results in increased reactivity of the kraft and/or sulfite dissolving pulp, particularly the kraft dissolving pulp having an increased reactivity of at least 10% (such as at least 20% or at least 30%).

The invention also relates to use of cellulase for treatment of unbleached or partially bleached or alkaline extracted dissolving pulp.

Cellulases

Step i) comprises use of one or more cellulases such as one or more cellulases described herein below.

Cellulases or cellulolytic enzymes are enzymes involved in hydrolysis of cellulose. In the hydrolysis of native cellulose, it is known that there are three major types of cellulase enzymes involved, namely cellobiohydrolase (1,4-β-D-glucan cellobiohydrolase, EC 3.2.1.91, e.g., cellobiohydrolase I and cellobiohydrolase II), endo-β-1,4-glucanase (endo-1,4-β-D-glucan 4-glucanohydrolase, EC 3.2.1.4) and β-glucosidase (EC 3.2.1.21).

In order to be efficient, the digestion of cellulose and hemicellulose requires several types of enzymes acting cooperatively. At least three categories of enzymes are necessary to convert cellulose into fermentable sugars: endo-glucanases (EC 3.2.1.4) that cut the cellulose chains at random; cellobiohydrolases (EC 3.2.1.91) which cleave cellobiosyl units from the cellulose chain ends and beta-glucosidases (EC 3.2.1.21) that convert cellobiose and soluble cellodextrins into glucose. Among these three categories of enzymes involved in the biodegradation of cellulose, cellobiohydrolases are the key enzymes for the degradation of native crystalline cellulose. The term "cellobiohydrolase I" is defined herein as a cellulose 1,4-beta-cellobiosidase (also referred to as exo-glucanase, exo-cellobiohydrolase or 1,4-beta-cellobiohydrolase) activity, as defined in the enzyme class EC 3.2.1.91, which catalyzes the hydrolysis of 1,4-beta-D-glucosidic linkages in cellulose and cellotetraose, by the release of cellobiose from the non-reducing ends of the chains. The definition of the term "cellobiohydrolase II activity" is identical, except that cellobiohydrolase II attacks from the reducing ends of the chains.

Endoglucanases (EC No. 3.2.1.4) catalyses endo hydrolysis of 1,4- beta -D-glycosidic linkages in cellulose, cellulose derivatives (such as carboxy methyl cellulose and hydroxy ethyl cellulose), lichenin, beta-1,4 bonds in mixed beta-1,3 glucans such as cereal beta-D-glucans or xyloglucans and other plant material containing cellulosic parts. The authorized name is endo-1,4- beta - D-glucan 4-glucano hydrolase, but the abbreviated term endoglucanase is used in the

present specification.

The cellulases may comprise a carbohydrate-binding module (CBM) which enhances the binding of the enzyme to a cellulose-containing fiber and increases the efficacy of the catalytic active part of the enzyme. A CBM is defined as contiguous amino acid sequence within a carbohydrate-active enzyme with a discreet fold having carbohydrate-binding activity. For further information of CBMs see the CAZy internet server (Supra) or Tomme et al., (1995) in Enzymatic Degradation of Insoluble Polysaccharides (Saddler, J.N. & Penner, M., eds.), Cellulose-binding domains: classification and properties. pp. 142-163, American Chemical Society, Washington.

In a preferred embodiment the cellulases may be a preparation as defined in co-pending application US application US 60/941,251. In a preferred embodiment the cellulase preparation comprising a polypeptide having cellulolytic enhancing activity (GH61A), preferably the one disclosed as SEQ ID NO:2 in WO 2005/074656. The cellulase preparation may further comprise a beta-glucosidase, such as the fusion protein disclosed in US 60/832,511. In an embodiment the cellulase preparation also comprises a CBH II, preferably *Thielavia terrestris* cellobiohydrolase II CEL6A. In an embodiment the cellulase preparation also comprises a cellulase enzymes preparation, preferably the one derived from *Trichoderma reesei.* In a preferred embodiment the cellulase preparation is Cellulase preparation A used in Example 1 and disclosed in co-pending US application US 60/941,251.

Cellulases are synthesized by a large number of microorganisms which include fungi, actinomycetes, myxobacteria and true bacteria but also by plants. Especially endoglucanases of a wide variety of specificities have been identified

The cellulase activity may, in a preferred embodiment, be derived from a fungal source, such as a strain of the genus *Trichoderma,* preferably a strain of *Trichoderma reesei;* a strain of the genus *Humicola,* such as a strain of *Humicola insolens;* or a strain of *Chrysosporium,* preferably a strain of *Chrysosporium lucknowense* or a strain of *Thielavia terrestris.*

Fungi and bacteria produces a spectrum of cellulolytic enzymes (cellulases) which, on the basis of sequence similarities (hydrophobic cluster analysis), can be classified into different families of glycosyl hydrolases [Henrissat B & Bairoch A; Biochem. J. 1993 293 781-788]. At present are known cellulases belonging to the families 5, 6, 7, 8, 9, 10, 12, 26, 44, 45, 48, 60, and 61 of glycosyl hydrolases.

Temperature used in step i):

The temperature used for step i) is typically from 20°C to 100°C such as a temperature interval selected from the group consisting of from 20°C to 30°C, from 30°C to 40°C, from 40°C to 50°C, from 50°C to 60°C, from 60°C to 70°C, from 70°C to 80°C, from 80°C to 90°C, from 90°C to 100°C, or any combination of these intervals.

Incubation time used in step i):

The incubation time used for step i) is typically from 5 minutes to 6 hours such as a time interval selected from the group consisting of from 5 minutes to 15 minutes, from 15 minutes to 30 minutes, from 30 minutes to 45 minutes, from 45 minutes to 60 minutes, from 1 hour to 1.5 hours, from 1.5 hours to 2 hours, from 2 hours to 2.5 hours, from 2.5 hours to 3 hours, from 3 hours to 3.5 hours, from 3.5 hours to 4 hours, from 4 hours to 4.5 hours, from 4.5 hours to 5 hours, from 5 hours to 5.5 hours, from 5.5 hours to 6 hours, or any combination of these time intervals.

Enzyme concentration used in step i):

The concentration (mg enzyme protein/ kg oven dry pulp) of the one or more cellulases used in step i) can in one embodiment be from 0.05 mg/kg oven dry pulp to 100 mg/kg oven dry pulp such as a concentration selected from the group consisting of from 0.05 mg/kg oven dry pulp to 0.25 mg/kg oven dry pulp, from 0.25 mg/kg oven dry pulp to 1.0 mg/kg oven dry pulp, from 1.0 mg/kg oven dry pulp to 5.0 mg/kg oven dry pulp, from 5.0 mg/kg oven dry pulp to 10.0 mg/kg oven dry pulp, from 10.0 mg/kg oven dry pulp to 15.0 mg/kg oven dry pulp, from 15.0 mg/kg oven dry pulp to 20.0 mg/kg oven dry pulp, from 20.0 mg/kg oven dry pulp to 30.0 mg/kg oven dry pulp, from 30.0 mg/kg oven dry pulp to 40.0 mg/kg oven dry pulp, from 40.0 mg/kg oven dry pulp to 60.0 mg/kg oven dry pulp, from 60.0 mg/kg oven dry pulp to 80.0 mg/kg oven dry pulp, and from 80.0 mg/kg oven dry pulp to 100.0 mg/kg oven dry pulp, or any combination of these intervals.

Bleaching in step ii):

The bleaching in step ii) can be performed by any conventional bleaching method including treatment with oxidizing agents such as chlorine (C-stage), chlorine dioxide (D-stage), oxygen (O-stage), hydrogen peroxide (P-stage), ozone (Z-stage) and peracetic acid (Paa-stage) or a reducing agent such as sodium dithionite (Y-stage). The bleaching can be done in one or more steps with washing in between them.

In a preferred embodiment the bleaching can be a chlorine ($Cl_2$; C-stage) free process such as the elemental

chlorine free (ECF) bleaching where chlorine dioxide ($ClO_2$; D-stage) is mainly used and typically followed by an alkaline extraction stage. Totally chlorine free (TCF) bleaching is another process where mainly oxygen-based chemicals are used.

Extraction (E) in step iii): is normally run using less than 2% odp NaOH at medium pulp consistency and temperature below 85°C. In general, the demand for caustic soda is normally lower than 1% odp for pulps of kappa number ca.10. This stage can be further supplemented with oxygen (Eo) or hydrogen peroxide (Ep) or both oxygen and hydrogen peroxide (Eop). A regular alkaline extraction stage (E) is tipically used to dissolve oxidized lignin from a previous chlorine dioxide stage in ECF bleaching while allowing further pulp oxidation and brightening with the co-addition of oxygen and hydrogen peroxide. It is typically followed by a washing stage before the next process step.

Cold Caustic Extraction (CCE) in step iii):

Cold caustic extraction (CCE) is a method used to to remove short-chain noncellulosic carbohydrates (cellulose purification) that is based on physical effects such as swelling and solubilization. Usually, a CCE stage takes place at temperatures below 45°C and using very high NaOH dosage that, in the liquid phase, can reach values up to 100 g/L. Depending on the pulp consistency in use, this will determine the amount of NaOH per dry weight of pulp. Typical conditions for a CCE-stage can be 5-10% w/w NaOH in the liquid phase for at least 10 min. It is typically followed by a washing stage before the next process step.

Hot Caustic Extraction (HCE) in step iii):

Hot Caustic Extraction (HCE) is a method to remove short chain hemicellulose and amorphous cellulose in dissolving pulps. In a (HCE)-stage the NaOH-concentration is not as high as in a cold alkali treatment, but the temperature is higher. It is typically followed by a washing stage before the next process step.

The temperature in HCE in step ii) is preferably from 70°C to 160°C. In a preferred embodiment the HCE temperature can be within a temperature interval selected from the group consisting of from about 70°C to about 75°C, from about 75°C to about 80°C,from about 80°C to about 85°C, from about 85°C to about 90°C, from about 90°C to about 95°C, from about 95°C to about 100°C, from about 100°C to about 105°C, from about 105°C to about 110°C, from about 110°C to about 115°C, from about 115°C to about 120°C, from about 120°C to about 125°C, from about 125°C to about 130°C, from about 130°C to about 135°C, from about 135°C to about 140°C, from about 140°C to about 145°C, from about 145°C to about 150°C, from about 150°C to about 155°C, and from about 155°C to about 160°C, or any combination of these intervals. If a temperature of 100°C or above 100°C is used the reaction is preferably performed at a pressure above atmospheric pressure such as at a pressure selected from the group consisting of pressure intervals from 1-2 bars, 2-3 bars, 3-4 bars, 4-5 bars, 5-6 bars, 6-7 bars, 7-8 bars, 8-9 bars or 9-10 bars or any combination of these intervals.

In a preferred embodiment the alkali source used in step ii) consists of or comprises NaOH. In another embodiment the alkali source used in step ii) consists of or comprises one or more alkali sources selected from the group consisting of NaOH $Ca(OH)_2$, $NH_4OH$ and $Mg(OH)_2$.

The hot caustic extraction in step ii) is in a preferred embodiment performed with an alkaline source (such as NaOH) at a concentration of less than 2 w/w %, such as less than 1.8 w/w %, such as less than 1.6 w/w %, such as less than 1.4 w/w %, such as less than 1.2 w/w %, such as less than 1.0 w/w %, such as less than 0.8 w/w %, such as less than 0.6 w/w %, such as less than 0.4 w/w %, such as less than 0.2 w/w %, or such as less than 0.15 w/w % (weight concentration of alkaline source in the liquid phase).

The hot caustic extraction in step ii) is in a preferred embodiment performed with an alkaline source (such as NaOH) consisting of or comprising hydroxide ions (such as NaOH) and the HCE is performed at a concentration of hydroxide ions of less than 1 M, such as less than 0.9 M, such as less than 0.8 M, such as less than 0.7 M, such as less than 0.6 M, such as less than 0.5 M, such as less than 0.4 M, such as less than 0.3 M, such as less than 0.2 M, such as less than 0.1 M, such as less than 0.09 M, such as less than 0.08 M, such as less than 0.07 M, such as less than 0.06 M, such as less than 0.05 M, such as less than 0.04 M, such as less than 0.03 M and such as less than 0.02 M.

The NaOH concentration used in the HCE in step ii) is typically less than 2 w/w %, such as less than 1.8 w/w %, such as less than 1.6 w/w %, such as less than 1.4 w/w %, such as less than 1.2 w/w %, such as less than 1.0 w/w %, such as less than 0.8 w/w %, such as less than 0.6 w/w %, such as less than 0.4 w/w %, such as less than 0.2 w/w %, or such as less than 0.15 w/w % (weight concentration of NaOH in the liquid phase).

The hot caustic extraction in step ii) is in a preferred embodiment performed with NaOH as the alkaline source and the HCE is performed at a concentration of NaOH of less than 1 M, such as less than 0.9 M, such as less than 0.8 M, such as less than 0.7 M, such as less than 0.6 M, such as less than 0.5 M, such as less than 0.4 M, such as less than 0.3 M, such as less than 0.2 M, such as less than 0.1 M, such as less than 0.09 M, such as less than 0.08 M, such as less than 0.07 M, such as less than 0.06 M, such as less than 0.05 M, such as less than 0.04 M, such as less than 0.03 M and such as less than 0.02 M.

The hot caustic extraction in step ii) is in a preferred embodiment performed with an alkaline source (such as

NaOH) at a concentration selected from the group consisting of from 0.1 w/w % to 0.2 w/w %, from 0.2 w/w % to 0.4 w/w %, from 0.4 w/w % to 0.6 w/w %, from 0.6 w/w % to 0.8 w/w %, from 0.8 w/w % to 1.0 w/w %, from 1.0 w/w % to 1.2 w/w %, from 1.2 w/w % to 1.4 w/w %, from 1.4 w/w % to 1.6 w/w %, from 1.6 w/w % to 1.8 w/w %, from 1.8 w/w % to 2.0 w/w %, or any combination of these intervals (weight concentration of alkaline source in the liquid phase).

The hot caustic extraction in step ii) is in a preferred embodiment performed with a NaOH concentration selected from the group consisting of from 0.1 w/w % to 0.2 w/w %, from 0.2 w/w % to 0.4 w/w %, from 0.4 w/w % to 0.6 w/w %, from 0.6 w/w % to 0.8 w/w %, from 0.8 w/w % to 1.0 w/w %, from 1.0 w/w % to 1.2 w/w %, from 1.2 w/w % to 1.4 w/w %, from 1.4 w/w % to 1.6 w/w %, from 1.6 w/w % to 1.8 w/w %, from 1.8 w/w % to 2.0 w/w %, or any combination of these intervals (weight concentration of alkaline source in the liquid phase).

The hot caustic extraction in step ii) is in a preferred embodiment performed with an alkaline source (such as NaOH) at a concentration of hydroxide ions selected from the group consisting of from 0.01 M to 0.025 M, from 0.025 M to 0.05 M, from 0.05 M to 0.1 M, from 0.1 M to 0.2 M, from 0.2 M to 0.3 M, from 0.3 M to 0.4 M, from 0.4 M to 0.5 M and from 0.5 M to 1 M, or any combination thereof.

The retention time for the HCE in step ii) is typically from 15 minutes to 5 hours. In a preferred embodiment the HCE retention time is within a time interval selected from the group consisting of from 15 minutes to 30 minutes, from 30 minutes to 45 minutes, from 45 minutes to 1 hour, from 1 hour to 1.5 hours, from 1.5 hour to 2 hours, from 2 hour to 2.5 hours, from 2.5 hour to 3 hours, from 3 hour to 3.5 hours, from 3.5 hour to 4 hours, from 4 hour to 4.5 hours, and from 4.5 hour to 5 hours, or any combination of these intervals.

Typical pulp consistencies used for the (HCE)-stage in step ii) is within the range between 2% and 30%. Preferably the pulp consistency used for the HCE in step ii) is from 5% to 20%, such as from 10% to 15%. In a preferred embodiment the pulp consistency used for HCE in step ii) is within an interval selected from the group consisting of from 2% to 4%, from 4% to 6%, from 6% to 8%, from 8% to 10%, from 10% to 12%, from 12% to 14%, from 14% to 16%, from 16% to 18%, from 18% to 20%, from 20% to 22%, from 22% to 24%, from 24% to 26%, from 26% to 28%, and from 28% to 30%, or any combination of these intervals.

Pulp used and produced in the method according to the invention:

The unbleached or partially bleached or alkaline extracted dissolving pulp used in the present invention is wood pulp coming from softwood trees (such as spruce, pine, fir, larch and hemlock) and/or hardwoods (such as eucalyptus, aspen and birch) or other plant sources such as bamboo.

In a preferred embodiment the unbleached or partially bleached or alkaline extracted dissolving pulp is selected from the group consisting of unbleached or partially bleached or alkaline extracted dissolving hardwood pulp and unbleached or partially bleached or alkaline extracted dissolving softwood pulp, or a mixture thereof.

In a preferred embodiment the hemicellulose content of the unbleached or partially bleached or alkaline extracted dissolving pulp produced according to the invention is less than 20% such as less than 15% or less 10%, such as less than 9%, such as less than 8%, such as less than 7%, such as less than 6%, such as less than 5%, such as less than 4%, such as less than 3%, such as less than 2% or such as less than 1%.

The invention relates in one embodiment to a kraft dissolving pulp made by the method according to the invention.

The invention relates in one embodiment to a sulfite dissolving pulp made by the method according to the invention.

The invention further relates to use of the dissolving pulp according to the invention for production of textile fibers. The dissolving pulp produced may be used in the manufacture of regenerated cellulose such as viscose rayon, lyocell and modal fibers.

The invention further relates to use of the dissolving pulp according to the invention for production of derivatized celluloses (cellulose derivatives) such as cellulose esters and ethers.

Performing the method of the invention in the presence of one or more surfactants

Step i) and/or step ii) and/or step iii) can be performed in the presence of one or more surfactants such as one or more anionic surfactants and/or one or more nonionic surfactants and/or one or more cationic surfactants.

Surfactants can in one embodiment include poly(alkylene glycol)-based surfactants, ethoxylated dialkylphenols, ethoxylated dialkylphenols, ethoxylated alcohols and/or silicone based surfactants.

Examples of poly(alkylene glycol)-based surfactant are poly(ethylene glycol) alkyl ester, poly(ethylene glycol) alkyl ether, ethylene oxide/propylene oxide homo- and copolymers, or poly(ethylene oxide- co-propylene oxide) alkyl esters or ethers. Other examples include ethoxylated derivatives of primary alcohols, such as dodecanol, secondary alcohois, poly[propylene oxide], derivatives thereof, tridecylalcohol ethoxylated phosphate ester, and the like.

Specific presently preferred anionic surfactant materials useful in the practice of the invention comprise sodium alpha-sulfo methyl laurate, (which may include some alpha-sulfo ethyl laurate) for example as commercially available

under the trade name ALPHA-STEP™-ML40; sodium xylene sulfonate, for example as commercially available under the trade name STEPA-NATE™-X; triethanolammonium lauryl sulfate, for example as commercially available under the trade name STEPANOL™-WAT; diosodium lauryl sulfosuccinate, for example as commercially available under the trade name STEPAN™-Mild SL3; further blends of various anionic surfactants may also be utilized, for example a 50%-50% or a 25%-75% blend of the aforesaid ALPHA-STEP™ and STEPANATE™ materials, or a 20%-80% blend of the aforesaid ALPHA-STEP™ and STEPANOL™ materials (all of the aforesaid commercially available materials may be obtained from Stepan Company, Northfield, Ill.).

Specific presently preferred nonionic surfactant materials useful in the practice of the invention comprise cocodiethanolamide, such as commercially available under trade name NINOL™-11CM; alkyl polyoxyalkylene glycol ethers, such as relatively high molecular weight butyl ethylenoxide-propylenoxide block copolymers commercially available under the trade name TOXIMUL™-8320 from the Stepan Company. Additional alkyl polyoxyalkylene glycol ethers may be selected, for example, as disclosed in U.S. Pat. No. 3,078,315. Blends of the various nonionic surfactants may also be utilized, for example a 50%-50% or a 25%-75% blend of the aforesaid NINOL™ and TOXIMUL™ materials.

Specific presently preferred anionic/nonionic surfactant blends useful in the practice of the invention include various mixtures of the above materials, for example a 50%-50% blends of the aforesaid ALPHA-STEP™ and NINOL™ materials or a 25%-75% blend of the aforesaid STEPANATE™ and TOXIMUL™ materials.

Preferably, the various anionic, nonionic and anionic/nonionic surfactant blends utilized in the practice of the invention have a solids or actives content up to about 100% by weight and preferably have an active content ranging from about 10% to about 80%. Of course, other blends or other solids (active) content may also be utilized and these anionic surfactants, nonionic surfactants, and mixtures thereof may also be utilized with known pulping chemicals such as, for example, anthraquinone and derivatives thereof and/or other typical paper chemicals, such as caustics, defoamers and the like.

## EXAMPLES

### Materials and Methods

Handsheets for measurement of "ISO brightness" (diffuse blue reflectance factor) were prepared according to ISO 3688 using a Büchner funnel and pressed with a Labtech automatic sheet press. The measurements were done using the Color Touch PC spectrophotometer from Technidyne. The intrinsic viscosity of the pulp was measured according to ISO 5351 and the TAPPI viscosity was measured according to TAPPI T230 procedure. The alkali solubility of pulp at 18% (w/w) NaOH (S18) was measured following the TAPPI procedure T 235. The content of pentosans in pulp was measured according to TAPPI procedure T 223.

The reactivity of pulp for viscose making was measured based on the known Fock's method described in the open literature. In this test, the pulp sample is dissolved in an excess of NaOH (9% w/w) and $CS_2$. A certain amount of dissolved cellulose reacts with $CS_2$. The excess $CS_2$ is then removed and cellulose is regenerated with $H_2SO_4$. The amount of cellulose that is regenerated with $H_2SO_4$ is determined by oxidation with potassium dichromate ($K_2Cr_2O_7$).

For the measurement of Fock's reactivity, 0.50 g of sample of pulp was added to 100 ml Erlenmeyer flask with a stopcock. 50 ml of NaOH (9% w/w) and 1 ml of $CS_2$ were added to the flask and the mixture was stirred for 3 hr with magnetic stirrer. The sample was transferred to a tube with stopcock, and distilled water was added to give a total weight of 100 g. The tube was shaken vigorously until the sample was well mixed. It was then centrifuged for 5 min at 6000 rpm. 10 ml of the liquid phase was pipetted into a 100 ml baker and neutralized with ca. 3 ml sulphuric acid (20% w/w). The mixture was set to react for 15-20 h, during which time the cellulose was regenerated and degassed to remove $CS_2$. 20 ml of $H_2SO_4$ (68 % w/w) was added and the sample was mixed and stirred for 1 h. The mixture was transferred to flask and 10 ml of 1/6 M $K_2Cr_2O_7$ was added; oxidation takes place whilst it was reboiled for 1 h. When the oxidized sample reached ambient temperature, it was poured into a 100 ml volumetric flask and 40 ml of the liquid was removed to react with an excess (5 ml) of potassium iodide (10 % w/w). The iodine produced was then titrated with sodium thiosulphate (0.1M) using starch as the indicator. The calculation of the pulp reactivity was based on the amount of unreduced $Cr^{6+}$ that remains after the oxidative reaction between potassium dichromate and cellulose which was converted into the amount of cellulose that has reacted with the carbon disulphide.

The enzymes used in the Examples are listed in the table herein below.

| Enzyme | SEQ ID NO in sequence listing |
|---|---|
| Cellulase from *Thielavia terrestris* | SEQ ID NO: 1 |
| Cellulase from *Humicola insolens* | SEQ ID NO: 2 |
| Cellulase from *Paenibacillus polymyxa* | SEQ ID NO: 3 |

(continued)

| Enzyme | SEQ ID NO in sequence listing |
|---|---|
| Xylanase from *Bacillus agaradhaerens* | SEQ ID NO: 4 |
| Xylanase from *Dictyoglomus thermophilum* | SEQ ID NO: 5 |

**Example 1: Enzymatic treatment of unbleached hardwood kraft pulp**

Two unbleached hardwood kraft dissolving pulps produced by a pre-hydrolysis kraft (PHK) pulping process were used and treated in an enzymatic-stage denoted as X-stage. Typically, 15 g of oven-dry fiber was treated with enzymes at medium pulp consistency of 10% at a temperature of 60°C, pH 5.5 (acetate buffer) for "pulp 1" or pH 8.0 (phosphate buffer) for "pulp 2" and for 90 min. The enzyme dosage was 0.050% odp of xylanase product from *Bacillus agaradhaerens* (SEQ ID NO: 4) and 0.025% odp of cellulase A product from *Humicola insolens* (SEQ ID NO: 2) and cellulase B product from *Paenibacillus polymyxa* (SEQ ID NO: 3).

The pulp suspension was incubated in polyethylene sealed plastic bags immersed in a temperature controlled water bath. After incubation, the pulp was washed and filtered with 2 L of warm tap water divided in two steps and 1 L of deionized water.

The intrinsic viscosity results of the produced pulps are presented in **Table 1.** Xylanases are frequently applied in pre-bleaching of kraft pulps to improve pulp bleachability (bleach boosting) but they are not expected to decrease pulp viscosity. As observed in **Table 1,** only when the cellulase is added it can be observed a drop in the pulp intrinsic viscosity.

**Table 1.** Pulp intrinsic viscosity after the enzymatic treatment (X-stage). The xylanase product used is from *Bacillus agaradhaerens* (SEQ ID NO: 4). The Cellulase A product is from *Humicola insolens* (SEQ ID NO: 2) and the Cellulase B product is from *Paenibacillus polymyxa* (SEQ ID NO: 3).

| Enzyme | Intrinsic viscosity (mL/g) | Variation vs. control (mL/g) |
|---|---|---|
| Pulp 1 | | |
| Original unbleached pulp 1 | 662 | --- |
| Control – no enzyme | 654 | 0 |
| xylanase | 663 | +9 (+1.4%) |
| xylanase + cellulase A | 522 | -132 (-20%) |
| xylanase + cellulase B | 593 | -61 (-9.3%) |
| Pulp 2 | | |
| Original unbleached pulp 2 | 1025 | --- |
| Control – no enzyme | 1005 | 0 |
| xylanase | 1010 | +5 (+0.5%) |
| xylanase + cellulase A | 725 | -280 (-28%) |
| xylanase + cellulase B | 867 | -138 (-14%) |

**Example 2: Full bleaching of the enzyme treated hardwood pulps and determination of final intrinsic viscosity and ISO brightness**

The hardwood kraft pulps produced in Example 1 were further treated by D-Ep-D-P bleaching sequence. The first D-stage was carried out at an initial pH of 3.5 and at 60°C for 1h using 0.25% (pulp 1) or 0.75% (pulp 2) odp $ClO_2$ while the second D-stage was at an initial pH of 4.0 and at ca. 75°C for 1.8 h and 0.16% (pulp 1) or 0.32% (pulp 2) odp of $ClO_2$. The Ep stage was carried out at an initial pH 11 and using 0.15% odp of $H_2O_2$, and 0.10% odp of $MgSO_4$ at ca. 75°C for 1.4h. As for the final P stage, the initial pH was 11 and 0.30% odp of $H_2O_2$, and 0.10% odp of $MgSO_4$ at ca. 80°C for 1.4h. All stages were done at 10% pulp consistency in polyethylene sealed plastic bags immersed in a temperature controlled water bath. After each stage, the pulp was washed and filtered as described in Example 1.

In **Table 2** is presented the intrinsic viscosity and ISO brightness of the produced X-D-Ep-D-P bleached dissolving pulps. When adding the xylanase alone, as in a typical bleach boosting application, it is observed an increase in the final brightness obtained and a slight increase in the intrinsic viscosity of the bleached pulp. When a cellulase is added together with a xylanase in the X-stage in the beginning of the bleaching sequence, there is a drop in the pulp viscosity while maintaining the higher ISO brightness compared to the control pulp.

**Table 2.** Pulp intrinsic viscosity and ISO brightness after X-D-Ep-D-P. The xylanase product used is from *Bacillus agaradhaerens* (SEQ ID NO: 4). The Cellulase A product is from *Humicola insolens* (SEQ ID NO: 2) and the Cellulase B product is from *Paenibacillus polymyxa* (SEQ ID NO: 3).

| X-stage ID | Intrinsic viscosity (mL/g) | Variation vs. control (mL/g) | ISO brightness (%) |
|---|---|---|---|
| Pulp 1 | | | |
| Control – no enzyme | 521 | 0 | 89.1 |
| xylanase | 540 | +19 (+3.6%) | 89.8 |
| xylanase + cellulase A | 438 | -83 (-16%) | 89.6 |
| xylanase + cellulase B | 500 | -21 (-4.0%) | 89.6 |
| Pulp 2 | | | |
| Control – no enzyme | 728 | 0 | 89.5 |
| xylanase | 741 | +13 (+1.8%) | 90.3 |
| xylanase + cellulase A | 595 | -133 (-18%) | 90.4 |
| xylanase + cellulase B | 663 | -65 (-8.9%) | 91.1 |

**Example 3: Correlation between the pulp viscosity obtained after the enzyme treatment done to the unbleached hardwood pulp and the final viscosity of the X-D-Ep-D-P fully bleached pulp**

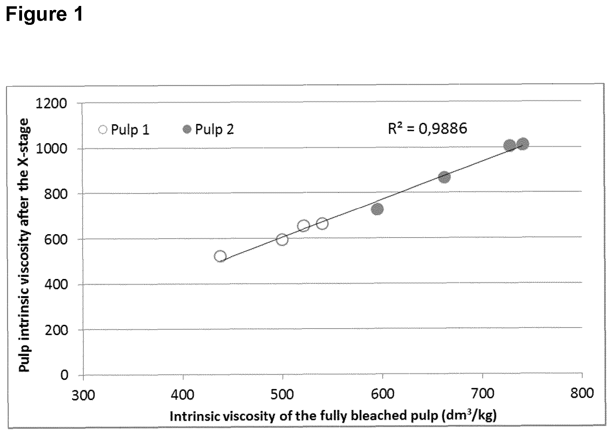

As seen in Figure 1, there is a noteworthy linear correlation between the pulp viscosity after the X-stage done in the beginning of the bleaching sequence as measured in Example 1 and the final viscosity of the fully bleached pulp as measured in Example 2. This reveals that the drop in pulp viscosity made by the cellulase in pre-bleaching is stable along the whole bleaching sequence and thus allowing higher flexibility with regard to the application point of the cellulase for the control of pulp viscosity and possibly increase of pulp reactivity. Although the extent of viscosity reduction between both

pulps is different, it is quite surprising to find in Figure 1 the same linearity for the two pulps which had originally different intrinsic viscosities and were treated at different pH (5.5 vs. 8.0) in the enzyme stage (X). This reveals that under the studied conditions while the enzyme was active, a reliable and consistent viscosity reduction by the use of a cellulase can be obtained regardless of the incoming pulp viscosity or operating conditions previously set.

**Example 4: Enzymatic treatment of unbleached and partially bleached mixed hardwood kraft pulp**

Unbleached and D-Ep partially bleached mixed hardwood (aspen+maple) kraft pulp produced by a pre-hydrolysis kraft (PHK) pulping process were used and treated in an enzymatic-stage denoted as X-stage. The enzymatic treatments with cellulase product from *Thielavia terrestris* (SEQ ID NO: 1) of both pulps at 10% consistency were carried out in a similar way as described in Example 1, at 60°C, pH 6.5 for different incubation times (15, 30, 60 and 90 min).

After 90 min of incubation time, the extent of the reduction of pulp viscosity differs depending on the dosage level and on the pulp type as shown in **Table 3.** Using the same operating conditions in the enzyme treatment of both pulps, a higher drop in pulp viscosity is obtained using the unbleached pulp which had initially a higher viscosity than the D-Ep partially bleached pulp at the three cellulase dosage levels.

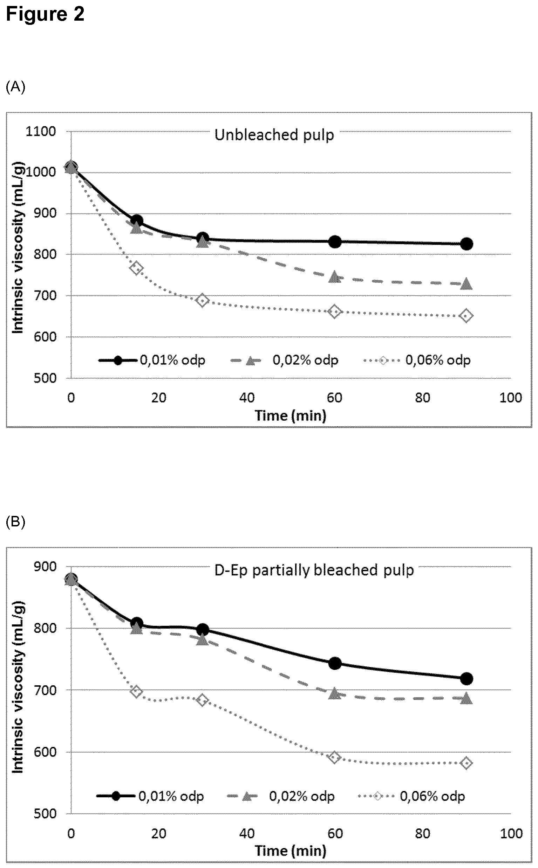

In terms of viscosity reduction profiles along time, it is seen in Figure 2 for both pulps that after ca. 60 min, a viscosity plateau is reached under the conditions studied. There is a steep viscosity reduction in the beggining which levels off after ca. 60 min. This viscosity plateau depends on the initial dosage level applied. This represents an important advantage compared to traditional chemicals applied for viscosity control such as NaOCl or oxygen-based chemicals because the cellulase can allow a selective and more controlled viscosity reduction without risking over-degradation of the cellulose. An additional trial under the same conditions except for a more moderate temperature of 50°C and using 0.06% odp cellulase product with the unbleached pulp showed that after 24h the plateau was the same as after 2h (final intrinsic viscosity of ca. 600 mL/g; ca. -40% reduction vs. original and control pulp)

**Table 3.** Pulp intrinsic viscosity of the original unbleached and D-Ep partially bleached pre-hydrolysed kraft pulps and after 90 min treatment with cellulase product from *Thielavia terrestris* (SEQ ID NO: 1) at three dosage (w/w) levels based on oven-dry pulp - odp.

| Enzyme | Intrinsic viscosity (mL/g) | Variation vs. control (mL/g) |
|---|---|---|
| Unbleached pulp | | |
| Original unbleached pulp | 1013 | --- |
| Control – no enzyme | 1037 | 0 |
| X: 0.01% odp cellulase | 826 | -211 (-20%) |
| X: 0.02% odp cellulase | 729 | -308 (-30%) |
| X: 0.06% odp cellulase | 651 | -386 (-37%) |
| D-Ep partially bleached | | |
| Original D-Ep pulp | 879 | --- |
| Control – no enzyme | 840 | 0 |
| D-Ep-X: 0.01% odp cellulase | 719 | -121 (-14%) |
| D-Ep-X: 0.02% odp cellulase | 687 | -153 (-18%) |
| D-Ep-X: 0.06% odp cellulase | 582 | -258 (-31%) |

Example 5: Two-step enzymatic treatment of mixed hardwood kraft pulp combined with bleaching

The same type of unbleached pulp as in Example 4 produced with a pre-hydrolysis kraft (PHK) cooking process having ISO brightness of 42.2% was treated with an enzymatic-stage (X1-stage) followed by chlorine dioxide (D-stage), then an alkaline extraction stage reinforced with hydrogen peroxide (Ep-stage) and then a second enzymatic-stage (X2-stage).

The X1-stage was carried out at 60°C for 45 min, pH 6.7 (adjusted with $H_2SO_4$) at 10% pulp consistency using xylanase (Xylanase product from *Dictyoglomus thermophilum;* SEQ ID NO: 5) and cellulase (Cellulase product from *Thielavia terrestris;* SEQ ID NO: 1) either combined or alone. The D-stage was performed at 3.5% consistency by diluting the X1-treated pulp (not washed) with water and chlorine dioxide solution. The dosage of chlorine dioxide in the D-stage varied between 1.2% $ClO_2$ odp (100% dosage; only used for one of the control samples without enzyme) and 1.0% odp (83.3% dosage; used for one control sample without enzyme and for all the enzyme treated samples) while the temperature was 60°C and the retention time 40 min. As for the alkaline extraction stage reinforced with hydrogen peroxide (Ep-stage), the operating conditions were: 70°C; 55 min; 0.9% NaOH odp; 0.16% $H_2O_2$, odp; 13% consistency. Finally, the second enzymatic stage (X2-stage) had only cellulase addition (Cellulase product from *Thielavia terrestris* with SEQ ID NO: 1) at three dosage levels (0.010, 0.015 and 0.020% odp) at pH 6.0, 50°C, for 100 min at 11% consistency.

After each stage, the pulp was washed as described in the previous examples with the exception of the first enzyme treatment where no washing was done before the D-stage being this fact represented by "X1/D".

The pulp viscosity was measured after X1/D-Ep and after X1/D-Ep-X2 treatment and following TAPPI T230 procedure while the ISO brightness was measured after X1/D-Ep.

The ISO brightness values measured after X1/D-Ep are presented in **Table 4.** When using the same $ClO_2$ dosage

in the D-stage (1.0% $ClO_2$ odp corresponding to 83.3% of normal dosage), all the enzyme treated pulps reach higher brightness compared to the corresponding control 2. A higher brightness is obtained when the xylanase was used even though the cellulase also led to an increased brightness when alone and therefore less $ClO_2$ will be required to reach the same brightness with the enzyme pre-treatment.

As regards pulp viscosity, this property was measured after X1/D-Ep and X1/D-Ep-X2 and the values are shown in **Table 5.** It can be seen a clear cellulase dosage profile in the extent of viscosity reduction allowing a reduction of -22% in X1-stage at 0.02% odp of cellulase product compared to the corresponding control (without enzyme). No effect of the xylanase product in viscosity reduction is observed once again.

Comparing unbleached pulp and X/D-Ep treated pulps having ca. 16 cP, the same dosage of cellulase when applied in X2 reduces more the viscosity than when applied in X1: -14% in X1 vs. -23% reduction in X2 (from 16.67 to 12.88 cP) at 0.015% odp cellulase; -22% in X1 vs. -27% reduction in X2 (from 16.67 to 12.10 cP) at 0.020% odp cellulase. This reveals that the degree of delignification impacts the performance of the cellulase regarding the extent of viscosity reduction and therefore using a partially bleached pulp a higher reduction is obtained compared to the unbleached pulp.

However, as a different pulp viscosity plateau is reached using unbleached or partially bleached pulp for the same dosage of cellulase product (shown in Example 4), it is preferable to split the dosages between two addition points (X1 and X2). The cellulase applied as 0.010+0.010 % odp reached -32% viscosity reduction (11.8 cP) in 2-stages (X1+X2), which is better than using 0.020% odp only in X1, which gave from -22%- to -18% of viscosity reduction.

Similarly to previous examples, a maximum reduction in pulp viscosity was obtained. Either using 0.035 or 0.040 %odp of total cellulase dosage, -40% was the maximum reduction in this pulp viscosity. This is an unique advantage of the use of endoglucanases to allow a precise control of pulp viscosity without a major risk of uncontrolled cellulose degradation that could lead to off-grade pulp (not meeting viscosity quality standards).

**Table 4.** ISO brightness of the X1/D-Ep treated pulps. Cellulase product from *Thielavia terrestris* (SEQ ID NO: 1) was applied at three dosage levels (w/w, based on oven dry pulp - odp): L = 0.010% odp; M = 0.015% odp; H = 0.020% odp. Xylanase product from *Dictyoglomus thermophilum* (SEQ ID NO: 5) was applied at three dosage levels: L = 0.0035% odp; M = 0.0070% odp; H = 0.0140% odp.

| Sample ID X1-stage conditions | ISO brightness (%) after X/D-Ep | |
|---|---|---|
| | Value (%) | Variation vs. Control 2 |
| Control 1 (no enzyme) D-stage with 100% $ClO_2$ | 79.8 | --- |
| Control 2 (no enzyme) D-stage with 83.3% $ClO_2$ | 77.8 | 0 |
| All enzyme treated pulps had 83.3% $ClO_2$ in the D-stage | | |
| X1: cellulase (M) | 78.3 | +0.5 |
| X1: cellulase (H) | 78.7 | +0.9 |
| X1: xylanase (L) | 79.4 | +1.6 |
| X1: xylanase (M) | 79.3 | +1.5 |
| X1: xylanase (H) | 78.6 | +0.8 |
| X1: cellulase (L) + xylanase (L) | 78.3 | +0.5 |
| X1: cellulase (L) + xylanase (M) | 79.0 | +1.2 |
| X1: cellulase (M) + xylanase (L) | 78.3 | +0.5 |
| X1: cellulase (M) + xylanase (M) | 78.2 | +0.4 |
| X1: cellulase (H) + xylanase (H) | 79.0 | +1.2 |

**Table** 5. TAPPI viscosity of the X1/D-Ep and X1/D-Ep-X2 treated pulps. Cellulase product with SEQ ID NO: 1 was applied at three dosage levels (w/w, based on oven dry pulp - odp): L = 0.010% odp; M = 0.015% odp; H = 0.020% odp. Xylanase product with SEQ ID NO: 5 was applied at three dosage levels: L = 0.0035% odp; M = 0.0070% odp; H = 0.0140% odp.

| X1/D-Ep treatment | | | X1/D-Ep-X2 treatment | | |
|---|---|---|---|---|---|
| Sample ID X1-stage conditions | TAPPI viscosity | | X2-stage Conditions | TAPPI viscosity | |
| | cP | Variation vs. Control 2 | | cP | Variation vs. Control 2 |
| Control 1 (no enzyme) D-stage with 100% $ClO_2$ | 14.48 | --- | Control 1 (no enzyme) | 14.88 | --- |
| Control 2 (no enzyme) D-stage with 83.3% $ClO_2$ | 16.33 | 0.00 | Control 2 (no enzyme) | 17.24 | 000 |
| All enzyme treated pulps had 83.3% $ClO_2$ in the D-stage | | | | | |
| X1 : cellulase (M) | 14.06 | -2.27 (-14%) | X2: cellulase (L) | 12.94 | -4.30 (-25%) |
| | | | X2: cellulase (M) | 10.87 | -6.37 (-37%) |
| X1: cellulase (H) | 12.73 | -3.60 (-22%) | X2: cellulase (L) | 11.70 | -5.54 (-32%) |
| | | | X2: cellulase (H) | 10.49 | -6.74 (-39%) |
| X1 : xylanase (L) | 18.36 | 2.04 | --- | --- | --- |
| X1 : xylanase (M) | 16.67 | 0.34 | X2: cellulase (M) | 12.88 | -4.36 (-25%) |
| | | | X2: cellulase (H) | 12.10 | -5.14 (-30%) |
| X1 : xylanase (H) | 16.28 | -0.05 | --- | --- | --- |
| X1 : cellulase (L) + xylanase (L) | 15.48 | -0.85 (-5%) | --- | --- | --- |
| X1: cellulase (L) + xylanase (M) | 14.26 | -2.07 (-13%) | X2: cellulase (L) | 11.79 | -5.45 (-32%) |
| | | | X2: cellulase (M) | 11.06 | -6.18 (-36%) |
| X1 : cellulase (M) + xylanase (L) | 13.26 | -3.07 (-19%) | --- | --- | --- |
| X1: cellulase (M) + xylanase (M) | 14.29 | -2.04 (-13%) | X2: cellulase (L) | 12.44 | -4.80 (-28%) |
| | | | X2: cellulase (M) | 11.14 | -6.10 (-35%) |
| X1: cellulase (H) + xylanase (H) | 13.32 | -3.01 (-18%) | X2: cellulase (L) | 11.42 | -5.82 (-34%) |
| | | | X2: cellulase (M) | 10.41 | -6.83 (-40%) |
| | | | X2: cellulase (H) | 10.36 | -6.88 (-40%) |

**Example 6: Effect of cellulase application in pre-bleaching (brownstock tower) on viscosity reduction, viscosity control, bleaching chemical savings and reactivity increase of the final dissolving pulp**

Cellulase product from *Thielavia terrestris* (SEQ ID NO: 1) was applied on a brownstock tower of a pre-hydrolysis kraft (PHK) hardwood dissolving pulp mill at a dosage level between 0.1-0.2 L/tonne of pulp. The bleaching sequence is D-Ep-H-Ep-D where the control of pulp viscosity is mainly done in the sodium hypochlorite stage (H-stage) and the conditions of each of the stages are standard as found in reference literature.

The enzyme product was added to the brownstock storage tank with an average pH ca. 5.7, temperature ca. 66°C at medium pulp consistency. At the target dosage of 0.2 L/tonne pulp of cellulase product that covered more than 4 days trial, the bleaching chemical consumptions were reduced, in particular sodium hypochlorite by 50% as compared to historical average which is presented in **Table 6.** This is because the cellulase product effectively decreased viscosity ca. 20 cP as measured after the D-stage compared to historical average before the trial.

In addition, the amount of off-grade pulp due to viscosity could be decrease from ca. 7.7% (month before the trial)

to ca. 1.0% during the trial at a dosage of 0.2 L/tonne pulp which corresponds to ca. 87% decrease in the production of such off-grade pulp. This important result reveals that the addition of the cellulase in the beginning of the bleaching sequence had a notable positive effect in the control of pulp viscosity along the bleaching process by reducing viscosity variation in the final bleached pulp. This translates into cost savings to the dissolving pulp mill.

Regarding the final parameters of the bleached dissolving pulp, **Table 6** shows that the S18 was reduced as well as the pentosans level which thus indicates a pulp of higher purity and therefore expected to have an improved processibility in the viscose making process or of any other production process of regenerated cellulosic fibers. The same improved quality can be seen in terms of Fock's reactivity in **Table 7** where the enzyme treated pulp shows a relative increase of 23% in pulp reactivity (Fock's method) which indicates higher reactivity in viscose making and therefore savings in chemicals and improved processability can be expected in the viscose making process.

**Table** 6. Bleaching chemical consumptions before and during the trial at 0.2L/tonne of cellulase product and purity of the dissolving pulp produced in terms of S18 and pentosans level.

| | NaOCl (kg/odmT) | $ClO_2$ (kg/odmT) | S18 (%) | Pentosans (%) |
|---|---|---|---|---|
| Previous 5 months average | 5,1 | 12,1 | 4,0 | 3,1 |
| Cellulase (0.2 L / tonne pulp) | 2,6 | 10,7 | 3,7 | 2,6 |
| Variation | -50% | -12% | -0,36 | -0,45 |

**Table 7.** Focks reactivity of the final dissolving pulp that was treated by X-D-Ep-H-Ep-D at 0.2L/tonne of cellulase product in the X-stage compared to D-Ep-H-Ep-D without enzyme addition.

| | 3 Days average | Day 4 |
|---|---|---|
| | No enzyme | Cellulase (0.2 L / tonne pulp) |
| Focks reactivity (%) | 45.7 | 56.2 |

**Example 7: Enzymatic treatment of unbleached and of HCE-treated softwood sulfite pulp**

An industrially produced unbleached sulfite softwood dissolving pulp was treated with an enzymatic-stage denoted as X-stage. The enzymatic treatments with cellulase product from *Thielavia terrestris* (SEQ ID NO: 1) at 10% pulp consistency were carried out in a similar way as described in Example 1, at 70°C, pH 6.0 (phosphate buffer 50 mM) for 60 min. The unbleached pulp which was not fully washed was further washed in the lab as described in Example 1 in order to compare the effect of washing efficiency on enzyme performance. The unbleached pulp was also submitted to a hot caustic extraction stage (HCE) at 10% consistency using 5% NaOH, 95C for 60 min to assess the performance of the cellulase on pulp viscosity reduction after HCE.

In **Table 8** it can be seen a modest decrease of pulp intrinsic viscosity after the enzyme treatment using the original unbleached pulp (ca. 9% reduction). The washing process in the lab improved the enzyme performance, due to negative effects of pulp carryover, allowing reaching a viscosity reduction of ca. 15%. Nevertheless, the reduction in pulp viscosity is inferior compared to previous examples using unbleached kraft pulp and the same cellulase product. However, a superior performance could be achieved when the cellulase was applied to the pulp after the alkaline HCE step, thus reaching a reduction of ca. 21%.

**Table 8.** Pulp intrinsic viscosity of the original unbleached pulp, with post lab washing or HCE treatment, before and after treatment with cellulase product from *Thielavia terrestris* (SEQ ID NO: 1) at two dosage levels expressed as mL of product per dry metric ton of pulp (mL/t odp).

| Enzyme | Intrinsic viscosity (mL/g) | Variation (mL/g) |
|---|---|---|
| Original unbleached pulp | 882 | 0 |
| X: 200 mL/t odp cellulase | 807 | -75 (-9%) |
| Washed pulp | 912 | 0 |
| X: 200 mL/t odp cellulase | 771 | -138 (-15%) |
| Original pulp after HCE | 964 | 0 |
| X: 200 mL/t odp cellulase | 760 | -207 (-21%) |

Another industrially produced sulfite softwood pulp after HCE treatment done in the pulp mill was additionally treated in the lab with an enzymatic-stage denoted as X-stage under the same conditions as before, except for the use of $H_2SO_4$ to adjust pH instead of buffer and a residence time of 120 min. As can be seen in **Table 9** the cellulase effectively reduced the viscosity by almost 30%. After the HCE treatment, the enzyme was thus very effective in viscosity reduction reaching similar reduction levels as seen previously for kraft pulps (e.g. Examples 4 and 5). In the case of sulfite pulps, a previous alkaline step as the HCE treatment before the cellulase treatment remarkably improves the performance of the cellulase in pulp viscosity reduction.

**Table 9** - Pulp intrinsic viscosity of the sulfite softwood pulp after HCE treatment, before and after treatment with cellulase product from Thielavia terrestris (SEQ ID NO: 1) at two dosage levels expressed as mL of product per dry metric ton of pulp (mL/t odp).

| Enzyme | Intrinsic viscosity (mL/g) | Variation (mL/g) |
|---|---|---|
| Original HCE treated pulp | 884 | --- |
| Control pH 7,4 | 892 | 0 |
| X: 200 mL/t odp cellulase (pH 7,3) | 660 | -232 (-26%) |
| X: 400 mL/t odp cellulase (pH 7,5) | 630 | -262 (-29%) |
| X: 200 mL/t odp cellulase (pH 5,6) | 644 | -248 (-28%) |

## Claims

**1.** A method for production of dissolving pulp with reduced viscosity comprising the steps of

i) treating partially bleached or alkaline extracted dissolving pulp with one or more cellulases (X stage) and
ii) bleaching of partially bleached/alkaline extracted pulp and
iii) optionally performing Alkaline Extraction of partially bleached/alkaline extracted pulp and
thereby generating dissolving pulp with reduced viscosity;
wherein the partially bleached or alkaline extracted dissolving pulp is wood pulp from softwood trees and/or hardwood trees; and
wherein step i) is performed prior to step ii).

**2.** The method according to claim 1 wherein the dissolving pulp is kraft pulp.

**3.** The method according to claim 1 wherein the dissolving pulp is sulfite pulp.

**4.** The method according to claims 1 to 3, wherein step ii) is performed using a chemical selected from the group consisting of $ClO_2$, $O_2$, $O_3$, $H_2O_2$, and NaOCl.

**5.** The method according to any of claims 1 to 4, wherein step iii) is an extraction, Hot Caustic Extraction or Cold Caustic Extraction stage.

**6.** The method according to any of claims 1 to 5, wherein the one or more cellulases used in step i) has a sequence identity of at least 60% (such as at least 65%, such as at least 70%, such as at least 75%, such as at least 80%, such as at least 85%, such as at least 90%, such as at least 95%, such as at least 99%) to SEQ ID NO: 1 or to SEQ ID NO:2 or to SEQ ID NO:3.

**7.** The method according to any of claims 1 to 6, wherein the one or more cellulases used in step i) is the cellulase having the amino acid sequence of SEQ ID NO: 1.

**8.** The method according to any of claims 1 to 7, wherein concentration expressed in mg enzyme protein/ kg oven dry pulp of the one or more cellulases used in step i) is from 0.05 mg/kg oven dry pulp to 100 mg/kg oven dry pulp such as a concentration selected from the group consisting of from 0.05 mg/kg oven dry pulp to 0.25 mg/kg oven dry pulp, from 0.25 mg/kg oven dry pulp to 1.0 mg/kg oven dry pulp, from 1.0 mg/kg oven dry pulp to 5.0 mg/kg oven dry pulp, from 5.0 mg/kg oven dry pulp to 10.0 mg/kg oven dry pulp, from 10.0 mg/kg oven dry pulp to 15.0 mg/kg oven dry pulp, from 15.0 mg/kg oven dry pulp to 20.0 mg/kg oven dry pulp, from 20.0 mg/kg oven dry pulp to 30.0 mg/kg oven dry pulp, from 30.0

mg/kg oven dry pulp to 40.0 mg/kg oven dry pulp, from 40.0 mg/kg oven dry pulp to 60.0 mg/kg oven dry pulp, from 60.0 mg/kg oven dry pulp to 80.0 mg/kg oven dry pulp, and from 80.0 mg/kg oven dry pulp to 100.0 mg/kg oven dry pulp, or any combination of these intervals.

**9.** The method according to any of claims 1 to 8, wherein the method results in improved viscosity control thereby reducing the production of off-grade pulp due to viscosity by more than 50% (such as more than 60% or more than 70%).

**10.** The method according to any of claims 1 to 9, wherein the method further comprises use of one or more xylanases and/or one or more mannanases and/or one or more lipases in step i).

**Patentansprüche**

**1.** Verfahren zur Herstellung von Chemiezellstoff mit verringerter Viskosität, umfassend die Schritte:

i) Behandeln von teilweise gebleichtem oder alkalisch extrahiertem Chemiezellstoff mit einer oder mehreren Cellulasen (X-Stufe), und
ii) Bleichen des teilweise gebleichten/alkalisch extrahierten Zellstoffs, und
iii) gegebenenfalls Durchführen einer alkalischen Extraktion des teilweise gebleichten/alkalisch extrahierten Zellstoffs, und
dadurch Erzeugen von Chemiezellstoff mit verringerter Viskosität;
wobei der teilweise gebleichte oder alkalisch extrahierte Zellstoff Holzstoff aus Nadelhölzern und/oder Laubhölzern ist; und
wobei Schritt i) vor Schritt ii) durchgeführt wird.

**2.** Verfahren nach Anspruch 1, wobei der Chemiezellstoff Kraftzellstoff ist.

**3.** Verfahren nach Anspruch 1, wobei der Chemiezellstoff Sulfitzellstoff ist.

**4.** Verfahren nach Ansprüchen 1 bis 3, wobei Schritt ii) unter Verwendung einer Chemikalie durchgeführt wird, die aus der Gruppe bestehend aus $ClO_2$, $O_2$, $O_3$, $H_2O_2$, und NaOCl ausgewählt ist.

**5.** Verfahren nach einem beliebigen der Ansprüche 1 bis 4, wobei Schritt iii) eine Extraktions-, Heißlaugextraktions- oder Kaltlaugextraktionsstufe ist.

**6.** Verfahren nach einem beliebigen der Ansprüche 1 bis 5, wobei die eine oder mehreren in Schritt i) verwendeten Cellulasen eine Sequenzidentität von mindestens 60% (wie mindestens 65%, wie mindestens 70%, wie mindestens 75%, wie mindestens 80%, wie mindestens 85%, wie mindestens 90%, wie mindestens 95%, wie mindestens 99%) zu SEQ ID NO: 1 oder zu SEQ ID NO: 2 oder zu SEQ ID NO: 3 aufweist.

**7.** Verfahren nach einem beliebigen der Ansprüche 1 bis 6, wobei die eine oder mehreren in Schritt i) verwendeten Cellulasen die Cellulase mit der Aminosäuresequenz von SEQ ID NO: 1 ist.

**8.** Verfahren nach einem beliebigen der Ansprüche 1 bis 7, wobei die Konzentration, ausgedrückt in mg Enzymprotein/kg ofentrockener Zellstoff, der einen oder der mehreren in Schritt i) verwendeten Cellulasen von 0,05 mg/kg ofentrockener Zellstoff bis 100 mg/kg ofentrockener Zellstoff ist, wie eine Konzentration ausgewählt aus der Gruppe bestehend aus von 0,05 mg/kg ofentrockener Zellstoff bis 0,25 mg/kg ofentrockener Zellstoff, von 0,25 mg/kg ofentrockener Zellstoff bis 1,0 mg/kg ofentrockener Zellstoff, von 1,0 mg/kg ofentrockener Zellstoff bis 5,0 mg/kg ofentrockener Zellstoff, von 5,0 mg/kg ofentrockener Zellstoff bis 10,0 mg/kg ofentrockener Zellstoff, von 10,0 mg/kg ofentrockener Zellstoff bis 15,0 mg/kg ofentrockener Zellstoff, von 15,0 mg/kg ofentrockener Zellstoff bis 20,0 mg/kg ofentrockener Zellstoff, von 20,0 mg/kg ofentrockener Zellstoff bis 30,0 mg/kg ofentrockener Zellstoff, von 30,0 mg/kg ofentrockener Zellstoff bis 40,0 mg/kg ofentrockener Zellstoff, von 40,0 mg/kg ofentrockener Zellstoff bis 60,0 mg/kg ofentrockener Zellstoff, von 60,0 mg/kg ofentrockener Zellstoff bis 80,0 mg/kg ofentrockener Zellstoff, und von 80,0 mg/kg ofentrockener Zellstoff bis 100,0 mg/kg ofentrockener Zellstoff, oder eine beliebige Kombination dieser Intervalle.

**9.** Verfahren nach einem beliebigen der Ansprüche 1 bis 8, wobei das Verfahren eine verbesserten Viskositäts-

steuerung zum Ergebnis hat, wodurch sich die Herstellung von Zellstoff minderer Qualität aufgrund der Viskosität um mehr als 50% (wie um mehr als 60% oder mehr als 70%) reduziert.

10. Verfahren nach einem beliebigen der Ansprüche 1 bis 9, wobei das Verfahren des Weiteren die Verwendung einer oder mehrerer Xylanasen und/oder einer oder mehrerer Mannanasen und/oder einer oder mehrerer Lipasen in Schritt i) umfasst.

## Revendications

1. Méthode pour la production de pâte dissolvante ayant une viscosité réduite, comprenant les étapes de

   i) traitement de pâte dissolvante partiellement blanchie ou extraite aux alcalis avec une ou plusieurs cellulases (stade X) et
   ii) blanchiment de la pâte partiellement blanchie/extraite aux alcalis et
   iii) éventuellement mise en œuvre d'une extraction aux alcalis de la pâte partiellement blanchie/extraite aux alcalis et
   ce qui génère ainsi de la pâte dissolvante ayant une viscosité réduite ;
   dans laquelle la pâte dissolvante partiellement blanchie ou extraite aux alcalis est de la pâte de bois provenant de conifères et/ou de feuillus ; et
   dans laquelle l'étape i) est effectuée avant l'étape ii).

2. Méthode selon la revendication 1, dans laquelle la pâte dissolvante est de la pâte kraft.

3. Méthode selon la revendication 1, dans laquelle la pâte dissolvante est de la pâte au sulfite.

4. Méthode selon les revendications 1 à 3, dans laquelle l'étape ii) est effectuée en utilisant un produit chimique choisi dans le groupe constitué par $ClO_2$, $O_2$, $O_3$, $H_2O_2$, et NaOCl.

5. Méthode selon l'une quelconque des revendications 1 à 4, dans laquelle l'étape iii) est une étape d'extraction, d'extraction caustique à chaud ou d'extraction caustique à froid.

6. Méthode selon l'une quelconque des revendications 1 à 5, dans laquelle la ou les cellulases utilisées dans l'étape i) ont une identité de séquence d'au moins 60 % (telle qu'au moins 65 %, telle qu'au moins 70 %, telle qu'au moins 75 %, telle qu'au moins 80 %, telle qu'au moins 85 %, telle qu'au moins 90 %, telle qu'au moins 95 %, telle qu'au moins 99 %) avec la SEQ ID NO : 1 ou avec la SEQ ID NO : 2 ou avec la SEQ ID NO : 3.

7. Méthode selon l'une quelconque des revendications 1 à 6, dans laquelle la ou les cellulases utilisées dans l'étape i) est la cellulase ayant la séquence d'acides aminés de la SEQ ID NO : 1.

8. Méthode selon l'une quelconque des revendications 1 à 7, dans laquelle la concentration, exprimée en mg de protéine enzymatique par kg de pâte séchée au four, de la ou des cellulases utilisées dans l'étape i), est de 0,05 mg/kg de pâte séchée au four à 100 mg/kg de pâte séchée au four, telle qu'une concentration choisie dans le groupe constitué par de 0,05 mg/kg de pâte séchée au four à 0,25 mg/kg de pâte séchée au four, de 0,25 mg/kg mg/kg de pâte séchée au four à 1,0 mg/kg de pâte séchée au four, de 1,0 mg/kg de pâte séchée au four à 5,0 mg/kg de pâte séchée au four, de 5,0 mg/kg de pâte séchée au four à 10,0 mg/kg de pâte séchée au four, de 10 mg/kg de pâte séchée au four à 15,0 mg/kg de pâte séchée au four, de 15,0 mg/kg de pâte séchée au four à 20,0 mg/kg de pâte séchée au four, de 20,0 mg/kg de pâte séchée au four à 30,0 mg/kg de pâte séchée au four, de 30,0 mg/kg de pâte séchée au four à 40,0 mg/kg de pâte séchée au four, de 40,0 mg/kg de pâte séchée au four à 60,0 mg/kg de pâte séchée au four, de 60,0 mg/kg de pâte séchée au four à 80,0 mg/kg de pâte séchée au four, et de 80,0 mg/kg de pâte séchée au four à 100,0 mg/kg de pâte séchée au four, ou n'importe quelle combinaison de ces intervalles.

9. Méthode selon l'une quelconque des revendications 1 à 8, dans laquelle la méthode a pour résultat un meilleur contrôle de la viscosité, ce qui ainsi réduit la production de pâte déclassée du fait d'une viscosité supérieure à 50 % (telle que supérieure à 60 % ou supérieure à 70 %).

10. Méthode selon l'une quelconque des revendications 1 à 9, dans laquelle la méthode comprend en outre l'utilisation d'une ou plusieurs xylanases et/ou d'une ou plusieurs mannanases et/ou d'une ou plusieurs lipases dans l'étape i).

## Figure 1

Pulp 1
Pulp 2
R² = 0,9886
1200
1000
800
600
400
200
0
300
400
500
600
700
800
Pulp intrinsic viscosity after the X-stage
Intrinsic viscosity of the fully bleached pulp (dm³/kg)

## Figure 2

(A)

Unbleached pulp
Intrinsic viscosity (mL/g)
1100
1000
900
800
700
600
500
0
20
40
60
80
100
Time (min)
0,01% odp
0,02% odp
0,06% odp

(B)

D-Ep partially bleached pulp
Intrinsic viscosity (mL/g)
900
800
700
600
500
0
20
40
60
80
100
Time (min)
0,01% odp
0,02% odp
0,06% odp

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2015107789 A
- US 60941251 B
- WO 2005074656 A
- US 60832511 B
- US 3078315 A


### Non-patent literature cited in the description


- Enzymatic Degradation of Insoluble Polysaccharides. **TOMME et al.** Cellulose-binding domains: classification and properties.. American Chemical Society, 1995, 142-163
- **HENRISSAT B** ; **BAIROCH A**. *Biochem. J.*, 1993, vol. 293, 781-788